Europäisches Patentamt

European Patent Office

Office européen des brevets

(19)

(11) Publication number : **0 611 263 A2**

(12) **EUROPEAN PATENT APPLICATION**

(21) Application number : **94830043.9**

(22) Date of filing : **07.02.94**

(51) Int. Cl.⁵ : **C12N 5/06**

(30) Priority : **10.02.93 IT RM930073**

(43) Date of publication of application :
**17.08.94 Bulletin 94/33**

(84) Designated Contracting States :
**AT BE CH DE DK ES FR GB GR IE LI LU MC NL PT SE**

(71) Applicant : **CELLIFE BIOTECNOLOGIE PER LA VITA S.r.l.**
**Via XX Settembre 28/8**
**I-16121 Genova (IT)**

(72) Inventor : **Moretta, Lorenzo**
**Viale Sacramentine, 3/16**
**I-16145 Genova (IT)**
Inventor : **Melioli, Giovanni**
**Via Polleri, 10/3**
**I-16125 Genova (IT)**

(74) Representative : **de Simone, Domenico et al**
**Ing. Barzanò & Zanardo Roma S.p.A.**
**Via Piemonte 26**
**I-00187 Roma (IT)**

(54) **A method to grow "in vitro" til cells.**

(57)    A method to grow in vitro TIL cell is described, comprising the steps as follows : - a single cell dissolution of a tumourous tissue ; - a said cell first incubation in a culture medium with a serum, that allows the lymphocyte growth, comprising interleukin 2 and phytolectin, for an interval from 3 to 5 days at 37°C in order to provide a primary culture ; - an isolation of mononucleated cell of said primary culture ; and - a second incubation of said monucleated cells in a culture medium, that allows the lymphocytes growth, comprising Interleukin-2, for an interval from 10 and 20 days at 37°C. Preferably, the method is used to grow TIL cells from NSSC.

EP 0 611 263 A2

Jouve, 18, rue Saint-Denis, 75001 PARIS

This invention relates to a method to cultivate TIL cells (tumour infiltrating lymphocytes), mainly from no small cell carcinomas (NSCC).

References, that in the follow will be indicated by numbers between brackets, are listed at the end of the description.

TIL cells, which may be detected close to solid tumours, consist of a T-enriched lymphocyte population, able to recognize tumour antigens, expressed on the cell surface of autologous neoplastic cells. A TIL cell sub-population shows a cytolytic activitiy on said autologous neoplastic cells, further to in vitro activation and grow in the presence of interleukin-2 (IL-2). Actually, it was recently found both for animals and humans, that a significant regression of some tumours, such as methastatic melanoma, could result following an intravenous administration of a large amount of in vitro grown, IL-2 activated autologous TIL cells. US patent 5.126.132 (Rosenberg) describes a method to treat some tumours by administering TIL cells to patients. However, the existing methods to in vitro cultivate and grow TIL cells, can be used only for lymphocyte populations derived from specific tumours, thus preventing the wider use of this therapy.

The lung NSCC is a most common neoplasia, that is partially insensitive to the chemiotherapy. Following a surgery removal and a supporting chemiotherapy, patients suffering NSCC tumours at IIIa or IIIb advanced stages, have an expected very short living time, i.e. 2 years approximatively, and only in 20% of cases. Accordingly, such patients are ideal candidates to be subjected to TIL cell immunotherapies. Hovever, only very recently TIL cells were evidentiated and isolated from NSCC tumours, though no positive results are kown in the art, as to attemps to in vitro grow TIL cells (1,2).

Accordingly, the need results to provide a method to cultivate and grow autologous TIL cells from tumours of various nature, and, specially, tumours with a strong social impact, such as NSCC. The authors make available a method to in vitro cultivate TIL cells, from lung NSCC tumours, which is able to supply a large amount of material, with a cost-contained procedure. They found that phytohaemoagglutinin (PHA), which is a well known T lymphocyte mitogen, could be used advantageously to selectively grow TIL cells from NSCC tumours, having a cytolytic activity, but not TIL cells from other tumours, such as melanomas.

Accordingly, it is an object of the invention a method to in vitro cultivate TIL cells, comprising the steps as follows:

- to dissociate a tumour tissue to single cells;
- to firstly incubate said cells in a serum containing culture medium, which is able to support the growth of lymphocytes, comprising IL-2 and PHA, for a time ranging from 3 to 5 days, at 37°C, in order to obtain a primary culture;
- to isolate mononucleated cells from said primary culture; and
- to secondly incubate said mononucleated cells again in a serum containing culture medium, which is able to support the growth of lymphocytes, comprising IL-2 for a time ranging from 10 to 20 days, at 37°C.

Preferably, said dissociating process comprises both a mechanical and an enzymatic dissociation, more preferably said enzymatic dissociation is carried out by incubating said tumour tissue, previously mechanically dissociated, with 0.002% of a solution of 100 UI/mg of DNAse, 200 UI/ml of collagenase, and 1500 UI/ml of hyaluronidase, for 3-5 hours at 37°C; said first incubation takes place with 500 UI/ml of IL-2 and 1% (w/v) of PHA; said mononucleated cell isolation occurs by treating said primary culture with a Ficoll-Hypaque gradient; and said second incubation is carried out at an IL-2 concentration of 500 UI/ml.

In a preferred embodiment, said tumour tissue is obtained from NSCC.

This invention now will be described by reference to some illustrative but not limiting embodiments.

## TISSUE SAMPLING

22 samples are picked up, under sterility conditions, from lung NSCC, from patients affected by this carcinoma at variable stages, from IIa, IIIa up to IIIb stage. Samples consist of appr. 50% of the surgery removed, tumoral and peritumural mass. Each sample is put in a sterility bag, partially filled with a RPMI culture medium (Gibco, U.S.), penicillin and streptomycin (1:100 v/v), as well as amphotericin B. The sample may be maintained for 24 hours at 4°C under the conditions as above.

After having mechanically removed some portions of the necrotic, adipose and apparently normal tissue, the tissue is mechanically dissociated, incubated under stirring with DNA-se (Sigma, USA, 0.002%, 100 UI/ml), type IV collagenase (Sigma, USA, 200 UI/ml), hyaluronidase (Sigma, USA, 50 U/ml), and gentamicin (16 mg/100 ml) for 3-5 hours at 37°C in a RPMI culture medium.

Isolated cells are collected and filtered on a mesh to remove not digested tissue fragments. In some cases, fragments are removed by using a Ficoll-Paque discontinuous gradient (Pharmacia, Sweden). The yield varied in the range from 40 to 3,200 millions of cells.

Staining with May-Grundwald-Giemsa shows a high percentage of neoplastic cells. The lymphocyte population ranges from 0.01% to 60% of whole cell amount. Results are summarized in table 1.

TABLE 1

| N | IN. | Rate | Percent of | | |
| | | | Tumour cells | Normal cells | Lymphocytes |
| --- | --- | --- | --- | --- | --- |
| 1 | MU | 1:10 | 50 | 30 | 20 |
| 3 | BE | 1:1000 | 50 | 50 | 0.01 |
| 4 | FR | n.c. | n.c. | n.c. | 2 |
| 5 | DE | n.c. | n.c. | n.c. | 0.05 |
| 6 | MA | n.c. | n.c. | n.c. | 0.01 |
| 7 | QU | n.c. | n.c. | n.c. | 0.01 |
| 10 | TO | n.c. | n.c. | n.c. | 2 |
| 12 | MA | n.c. | n.c. | n.c. | 5 |
| 13 | SO | 1:100 | 75 | 20 | 5 |
| 15 | AL | 1:250 | 50 | 25 | 10* |
| 16 | NA | 1:500 | 80 | 15 | 5 |
| 17 | BE | 1:50 | 15 | 40 | 30* |
| 18 | TU | 1:10 | 10 | 30 | 60 |
| 19 | GA | 1:200 | 5 | 90 | 5 |
| 20 | ST | 1:70 | 85 | 2 | 13 |
| 21 | GI | 1:250 | 70 | 27 | 3 |
| 22 | CO | 1:20 | 5 | 75 | 20* |

In. = patient's code; n.c. = not calculated; * = plus granulocites. Lymphocyte and erythrocyte % are calculated after a visive exhamination; normal cell (comprising fibroblasts and macrophages), tumor cells and lymphocytes are calculated after staining.

Isolated cells are washed with RPMI 1640 culture medium and incubated into complete medium (CM) at $5 \times 10^5$ cell/ml. CM comprises RPMI 1640 with 10% of human AB serum or autologous serum, 500 UI/ml of recombinant IL-2 (rIL-2, Proleukin, Cetus). PHA is added at a concentration of 1% by volume (PHA, Difco) to the assayed samples.

Cultures are incubated in flasks of 175 cm$^2$, for 3-5 days at 37°C. Further cells are repeatedly washed out with RPMI culture medium and mononucleated living cells are isolated using a Ficoll-Hypaque discontinuous gradient (Pharmacia, Sweden). Cells, comprising both lymphocytes and tumour cells, are then incubated in CM at $1-2 \times 10^6$ cells per ml, with 500 UI/ml of IL-2. Some samples are previously incubated with 1% of PHA in a IL-2-free CM for two days. When cells reach $3-4 \times 10^6$ per ml, cultures are diluted in the same media at 1:2 ratio and then incubated again. A substantially lymphocyte free population is obtained within 25 days from success cultures. TIL cells cultures may be propagated using a method as above whenever an original tissue comprises lymphocytes. The growing rate ranges from 100 to 200 times in PHA treated cultures, and from 50 to 200 times in PHA not treated cultures.

## SURFACE PHENOTYPE OF CULTIVATED LYMPHOCYTES

Cultures are submitted to immunofluorescence and flow cytofluorometry assays. In order to detect membrane receptors, that are indicative of a specific activation stage, the following monoclonal antibodies are used: anti-CD3 (leu-4, Becton Dickinson), anti-CD4, anti-CD8 and anti-CD19 (T4, T8 and B4 respectively, Coulter Sci.), anti-CD16 (KDL, 3), anti-CD25 (4), anti-HLA-DR (4), FITC labelled anti-human IgG (Southern Biol. Ass.).

0.05 ml of a lymphocyte culture are incubated with a previously titered antibody solution for 30 minutes at 4°C, then washed out and incubated again with 0.05 ml of antibodies anti-IgG for 30 minutes at 4°C. Ccells are washed out repeatedly and submitted to a flow cytofluorometry assay, using an RPICS Elite cytofluorometer.

All cultures of lymphocytes react positively with anti-CD3; a varying population, ranging from 18% to 88% react positively with anti-CD4, and a varying population, ranging from 14% to 82% react positively with anti-CD8. Results of the immunophenotyping are summarized at the table 2 herebelow.

## TABLE 2

| N. | In. | days cult. | lymph. tot. | CD3 % | CD4 % | CD8 % | CD16 % | Ig % | DR % | CD25 % |
|---|---|---|---|---|---|---|---|---|---|---|
| 1 | MU | 6 | 40 | 96 | 88 | 9 | 2 | 0 | NC | NC |
|  |  | 18 | 3300 | 92 | 30 | 48 | 0 | 0 | NC | NC |
| 3 | BE | 27 | 40 | 97 | 23 | 82 | 0 | 0 | NC | NC |
| 4 | FR | 18 | 288 | 76 | 27 | 34 | 3 | 0 | NC | NC |
|  |  |  | 300 | 92 | 22 | 69 | 8 | 0 | 75 | 11 |
| 5 | DE | 15 | 800 | 70 | 43 | 13 | 2 | 0 | NC | NC |
|  |  |  | 6000 | 95 | 75 | 14 | 2 | 0 | 61 | 24 |
| 6 | MA | 21 | 1000 | 96 | 18 | 54 | 1 | 0 | NC | NC |
|  |  |  | 2000 | 95 | 33 | 65 | 1 | 0 | 51 | 15 |
| 7 | QU | 30 | 50 | 96 | 65 | 34 | 3 | 0 | NC | NC |
|  |  | 45 | 100 | 95 | 45 | 60 | 4 | 0 | 72 | 48 |
| 8 | CH | 10 | 100 | 96 | 55 | 40 | 2 | 0 | 41 | 6 |
| 12 | MA | 15 | 1000 | 96 | 18 | 54 | 0 | 0 | NC | NC |
|  |  | 30 | 2500 | 87 | 20 | 40 | 0 | 0 | 46 | 6 |
| 13 | SO | 15 | 800 | 96 | 46 | 51 | 2 | 0 | NC | NC |
|  |  | 27 | 40000 | 88 | 34 | 48 | 2 | 0 | 35 | 7 |
| 14 | MI | 15 | 4000 | 68 | 24 | 47 | 4 | 0 | 51 | 31 |
| 15 | AL | 20 | 1500 | 65 | 14 | 42 | 0 | 0 | 52 | 3 |
| 16 | NA | 8 | 90 | 82 | 45 | 34 | 4 | 0 | 80 | 21 |
| 17 | BA(a) | 10 | 700 | 85 | 6 | 79 | 2 | 0 | 74 | 8 |
|  | BE(b) | 10 |  | 88 | 3 | 80 | 0 | 0 | 0 | 0 |
| 18 | TU | 28 | 5000 | 88 | 9 | 80 | 3 | 0 | 78 | 6 |
| 19 | GA(a) | 12 | 3000 | 61 | 18 | 41 | 4 | 0 | 60 | 3 |
|  | GA(b) | 12 |  | 90 | 40 | 42 | 2 | 0 | 75 | 20 |
| 20 | ST(a) | 8 | 3500 | 70 | 53 | 26 | 3 | 0 | 70 | 65 |

|    |        |    |       | NC | NC | NC | NC  | NC | NC | NC |
|----|--------|----|-------|----|----|----|-----|----|----|----|
|     | ST(b)  | 8  |       |    |    |    |     |    |    |    |
| 21 | CI(a)  | 30 | 18000 | 93 | 22 | 60 | 1,4 | 0  | 84 | 46 |
|     | CI(b)  | 30 |       | 91 | 56 | 32 | 5,7 | 0  | 83 | 28 |
| 22 | CO(a)  | 7  | 330   | 80 | 21 | 57 | 6,5 | 0  | 49 | 41 |

In. = patient's code; days colt. = culture day when the test is carried out; lymph. tot. = lymphocyte number at the test performance; CD3 = T lymphocytes; CD4 = T helper lymphocytes; CD8 = cytotoxic lymphocytes; CD16 = "natural killer" cells; Ig = B positive lymphocytes for surface IgG's; DR = B and T activated lymphocytes expressing IL-2 receptor; n.c. = not calculated.

CYTOLYTIC ACTIVITY

The cytolysis activity is analized on a number of target cells. The natural cytolytic activity (NK, natural killer) is evaluated using K562 cells (5); the lymphokine activated cytolytic activity (LAK) using NK resistant Daudi cells (6). On the contrary, the cytolytic specific activity is evaluated on autologous tumour cells, isolated and cultivated as indicated herebelow.

Single cell suspension is obtained through mechanical and enzymatic separation of neoplastic tissue, as previously described. Cells are plated on a single layer of murine 3T3J2 fibroplasts, that are letally irradiated, in a culture medium, comprising DMEM and Ham's culture media (Gibco), containing, at 2:1 ratio, 10% of FCS, 5 µg/ml of insulin, 5 µg/ml of transferrin, 0.4 mg/ml of hydrocortisone, 0.1 nM of cholera toxin, 20 pM of triiodothyronine; 50 µg/ml of penicillin and 50 µg/ml of streptomycin. 10 mg/ml of Epidermal Growth Factor (EGF) are added after two days. The presence of neoplastic cells is detected through cytology and/or immunofluorescence tests. Cells are stored by freezing, using liquid nitrogen.

Cells are labelled by $^{51}CrNa_2O_4$ (100 ml/$10^6$ cells) (4). Effector/target cells, diluted from 50:1 to 55:1 concentrations are incubated for 4 hours at 37°C. The lysis units, calculated as the amount of effector cells per $10^6$ cells, that is requested to lysate the 30 percent of 5000 target cells, are calculated in respect of the NK activity. The specific lysis is calculated, as described in (4).

Most of the samples of the in vitro grown cell with IL-2 show a powerful NK on K652 cells. Highest activity levels are obtained with not PHA treated cultures.

An evident cytolytic activity on NK-resistant Daudi cells is detected on not PHA treated lymphocytes, and also on PHA treated lymphocytes, though at lower levels.

The results are summarized at Table 3.

Table 3

| N. | In. | K562 | Daudi | tumor autologous cells | allogenic cells |
|---|---|---|---|---|---|
| 1 | MU | 7 | 10 | NS | NS |
| 3 | BE | 44 | 32 | NS | NS |
| 4 | FR | <5 | 38 | NS | NS |
| 5 | DE | 13,8 | 7 | 9 | 0 |
| 6 | NA | <5 | 5 | 10 | 0 |
| 7 | QU | <5 | 17 | NS | 0 |
| 8 | CH | 24 | 0 | NS | 19 |
| 12 | MA | 22 | 5 | NS | 9 |
| 13 | SO | 6,2 | 10 | 13 | 0 |
| 14 | MI | 21 | NS | NS | 5 |
| 15 | AL | 45 | 5 | NS | 10 |
| 16 | NA | 12 | 3 | 1 | 0 |
| 17 | BE(a) | 66 | 3 | NS | 0 |
|  | BE(b) | 70 | 28 | NS | 0 |
| 18 | TU | 28 | 12 | NS | 2 |
| 19 | GA(a) | 26 | 14 | 41 | 0 |
|  | GA(b) | 48 | 42 | 36 | 0 |
| 20 | ST(a) | 40 | 21 | 0 | 0 |
|  | ST(b) | 51 | 16 | 0 | 0 |
| 21 | CI(a) | 38 | 11 | 8 | 0 |
|  | CI(b) | 63 | 28 | 9 | 0 |
| 22 | CO(a) | 30 | 10 | 16 | 0 |
|  | CO(b) | 32 | 16 | 14 | 1 |

K562 = cytolysis activity (in U.L./$10^6$ cells) on K562 cells; Daudi = Cytol. act. (in lysis % at 40:1 E/T) on NK-resistant Daudi cells; autologous tumour cells = cytolysis act. (in lysis % at 40:1 E/T) on short term cultures of lung carcinoma; allogenic tum. cells = cytolysis act. (in lysis % at 40:1 E/T) on short term cultures of allogenic tum. cells; n.s. = not assayed; (a) = plus PHA; (b) = less PHA

By testing the cytolytic activity on autologous or allogenic tumour cells no differences result between the PHA treated and PHA not treated lymphocytes, thus showing that the cytolysis potential is not limited to the CD25+ lymphocyte population, already in vivo activated, which is 10-20% of total lymphocytes. Also the PHA activated, lymphocyte population, that is 80-90% of total lymphocytes, show a cytolytic activity on autologous tumour cells.

REFERENCES

1. Yano, T. and al., "Int. J. Cancer", 48, 619-623, 1989
2.Viale, M. and al., "Tumouri", 76(5), 488-494, Oct.31, 1990
3.Moretta, A. and al., "J. Exp. Med.",157, 743-750, 1983
4.Moretta, A. and al., "Int. J. Cancer", 44, 124-132, 1989
5. Trinchieri, G. and Perussia, B., " Lab. Invest.", 50, 489
6. Moretta, A. and al., "Europ. J. Immunol.", 102, 572.

**Claims**

1. A method to <u>in vitro</u> cultivate TIL cells, comprising the steps as follows:
   - to dissociate a tumour tissue to single cells;
   - to firstly incubate said cells in a serum containing culture medium, which is able to support the growth of lymphocytes, comprising IL-2 and PHA, for a time ranging from 3 to 5 days, at 37°C, in order to obtain a primary culture;
   - to isolate mononucleated cells from said primary culture; and
   - to secondly incubate said mononucleated cells again in a serum containing culture medium, which is able to support the growth of lymphocytes, comprising IL-2 for a time ranging from 10 to 20 days, at 37°C.

2. A method to <u>in vitro</u> cultivate TIL cells according to claim 1, wherein said dissociation comprises a mechanical and an enzymatic dissociation; most preferably said enzymatic dissociation is carried out through incubating said dissociated tumourous tissue with 0.002% of 100 UI/mg DNA-se; 200 UI/ml of collagenase and 1500 UI/ml of hyaluronidase for 3-5 hours at 37°C; said first incubation is carried out at an IL-2 concentration of 500 UI/ml and a PHA concentration of 1% w/v; said mononucleated cell isolation is carried out through treating said primary culture on a Ficoll-Hypaque dicontinuous gradient; and said second incubation is carried out at a 500 UI/ml concentration of IL-2.

3. A method to <u>in vitro</u> cultivate TIL cells according to any previous claim, wherein said tumour tissue is obtained from NSCC.